# DEMANDE DE BREVET EUROPEEN

(11) **EP 1 958 614 A1**
(43) Date de publication de la demande: **20.08.2008**
(21) Numéro de dépôt: 08101380.7
(22) Date de dépôt: 07.02.2008
(51) Int. Cl.: A61K 8/92, A61K 8/97, A61Q 19/00, A61Q 3/00, A61Q 19/08

(54) **Composition comprenant de l'huile d'argan et un produit à base de plante du genre aloe, et son utilisation cosmétique**

(30) Priorité: 14.02.2007 MA 29681; 14.05.2007 FR 0755045
(71) Demandeur: Alleon, Philippe, Inezgane (MA)
(72) Inventeur: Alleon, Philippe, Inezgane (MA)
(74) Mandataire: Domenego, Bertrand

(57) **Abrégé**

L'invention concerne une composition comprenant de l'huile d'argan et un extrait d'*Aloe ;* son utilisation pour fabriquer une formulation cosmétique et ladite formulation cosmétique en résultant.

## Description

L'invention concerne une composition à usage cosmétique et ses applications.

Le genre *Aloe* (division des Magnoliophyta, classe des Liliopsida, ordre des Asparagales, famille des Asphodelaceae) comprend environ 400 espèces de plantes succulentes à fleurs communément appelées aloès dont la plus connue est sûrement l'*Aloe vera.* La plupart des aloès présentent une rosette de feuilles larges, épaisses et piquantes en forme de lance. Ils paraissent souvent sans tige, la rosette poussant directement sur le sol.
Il est connu d'utiliser des produits dérivés de *l'Aloe vera* dans des préparations cosmétiques.

L'huile d'argan est extraite des fruits de l'arganier *(Argania spinosa,* famille des Sapotacées), les amendons. Cet arbre est peu connu puisqu'il pousse uniquement dans la partie sud-ouest du Maroc sur une superficie de 700 000-800 000 hectares.
Les fruits de l'arganier sont verts. Ils ressemblent à une olive, mais sont plus gros et plus ronds. À l'intérieur, se trouve une noix dont la coquille est très dure. Celle-ci représente environ un quart du poids du fruit. La noix peut contenir jusqu'à trois amendons à partir desquels sera extraite l'huile d'argan par pression mécanique à froid.
Il est connu que l'huile d'argan peut être utilisée dans des préparations cosmétiques.
La demande de brevet FR2553788 décrit un procédé de préparation d'un extrait lipidique de fruit d'arganier.
La demande de brevet FR2756183 décrit une composition pharmaceutique ou cosmétique comprenant une combinaison d'huile d'argan et de peptides d'argan.
La demande WO 01/37792 décrit des compositions dermatologiques comprenant de l'amidon de riz, des produits de coco, du beurre de karité, de l'huile de bourrache, de l'huile d'avocat, de l'huile de jojoba, et éventuellement, 1,5% en poids d'huile d'argan.
La demande de brevet EP1764085 décrit une composition cosmétique comprenant au moins 10% en poids d'huile d'argan.
Le Demandeur a maintenant découvert, de manière surprenante, que l'association d'huile d'argan et d'un produit à base de plante du genre *Aloe* permet d'obtenir une activité très supérieure à celle de l'huile d'argan seule ou à celle dudit produit à base de plante du genre *Aloe* seul.

Sur la base de cette découverte, un but de l'invention est de fournir une composition destinée à une utilisation cosmétique.

Un deuxième but de l'invention est de fournir des formulations cosmétiques destinées à une application topique.

Ainsi, selon un premier aspect, la présente invention a pour objet une composition comprenant de l'huile d'argan et un produit à base de plante du genre *Aloe.*
Dans le cadre de la présente invention, on entend par "produit à base de plante du genre *Aloe"* un produit dérivé d'une plante du genre *Aloe,* par exemple un jus, une pulpe, un extrait, une huile, un lyophilisat, un produit de zéodratation, un produit de déshydratation ou un mélange de ceux-ci obtenu(e) à partir d'un organe, d'un liquide ou d'un broyat provenant directement d'une plante du genre *Aloe.* De préférence, ce produit dérivé est obtenu à partir des feuilles.
Dans le cadre de la présente invention, on entend de préférence par « plante du genre *Aloe* » l'*Aloe vera* ou *l'Aloe arborescens,* de manière particulièrement préférée *l'Aloe arborescens.*
Avantageusement, l'huile d'argan est présente dans la composition selon l'invention à raison de 10% à 40%, de préférence 20% à 30%, en particulier 25% en poids de la composition.
Avantageusement encore, le produit à base de plante du genre *Aloe* est présent à raison de 10% à 40%, de préférence de 20% à 30%, en particulier 25% en poids de la composition.
Il faut bien souligner que c'est spécifiquement l'utilisation combinée d'huile d'argan et d'un produit à base de plante du genre *Aloe* qui mène à l'effet observé avec la composition selon l'invention. Cette combinaison a un effet nettement supérieur à celui de l'huile d'argan seule ou du même produit à base de plante du genre *Aloe* seul, notamment un effet nourrissant en application topique sur les cheveux, la peau ou les ongles.
Les compositions selon l'invention peuvent comprendre, en plus de l'huile d'argan, une ou plusieurs autres huiles végétales, de préférence choisies dans le groupe constitué de huile d'olive, huile d'avocat, huile de karité, huile de jojoba.

Les compositions selon l'invention peuvent comprendre en tant que composant supplémentaire un extrait de noni (Morinda citrifolia).

Un procédé de préparation d'une composition selon l'invention est un objet supplémentaire de la présente invention.
Dans un tel procédé de préparation, on mélange de l'huile d'argan et un produit à base de plante du genre *Aloe* pour obtenir une émulsion.

Un autre objet de la présente invention est l'utilisation des compositions selon l'invention pour préparer des formulations cosmétiques. De telles formulations cosmétiques font également partie de l'invention.

De façon à assurer un effet optimal au sein de la formulation cosmétique dans laquelle elle est incorporée, la composition selon l'invention est généralement mise en oeuvre dans ladite formulation à raison de 0,1 à 100% en poids, de préférence à raison de 30% à 40% en poids, de manière particulièrement préférée à raison de 40% à 50% en poids.
Les formulations cosmétiques selon l'invention peuvent comprendre, en plus des compositions selon l'invention, un ou plusieurs véhicules cosmétiquement acceptables.
De préférence, les formulations cosmétiques selon l'invention peuvent comprendre, en tant que véhicules cosmétiquement acceptables, l'eau, un solvant organique miscible à l'eau, des huiles naturelles, acides gras naturels ou cires naturelles, ou des mélanges de ceux-ci.

Par « véhicule cosmétiquement acceptable », on entend un véhicule adapté pour une utilisation en contact avec des cellules humaines et animales, en particulier les cellules de l'épiderme, sans toxicité, irritation, réponse allergique indue et similaire, et proportionné à un rapport avantage/risque raisonnable.
Les formulations cosmétiques selon l'invention peuvent comprendre, en plus des compositions selon l'invention, un ou plusieurs autres additifs cosmétiquement acceptables, choisis de préférence dans le groupe constitué de excipients, émulsifiants, tensioactifs, agents de conservation, parfums, colorants, pigments, stabilisants.

De telles formulations cosmétiques selon l'invention se présentent généralement sous forme de lotion, d'émulsion fluide, de crème, de lait, de mousse, de gel, d'aérosol ou de patch.
Il est des compétences de l'homme du métier de déterminer la nature du ou des composant(s) supplémentaire(s) à mettre en oeuvre dans les formulations de l'invention compte tenu du type de formulation cosmétique qu'il souhaite obtenir.

Un autre objet de l'invention est l'utilisation d'une formulation cosmétique selon l'invention en application topique pour nourrir, revitaliser et/ou raffermir la peau.
Un autre objet de l'invention est l'utilisation d'une formulation cosmétique selon l'invention en application topique pour nourrir et/ou revitaliser les cheveux.
Un autre objet de l'invention est l'utilisation d'une formulation cosmétique selon l'invention en application topique pour nourrir et/ou revitaliser les ongles.
Un autre objet de l'invention est l'utilisation d'une formulation cosmétique selon l'invention en application topique sur la peau à activité anti-âge et/ou anti-rides.
Un autre objet de l'invention est l'utilisation d'une formulation cosmétique selon l'invention en application topique sur la peau pour atténuer les taches de vieillesse.

De préférence, la formulation cosmétique selon l'invention est utilisée en application topique sur le visage et/ou les mains.

Les exemples suivants illustrent l'invention sans en limiter la portée.

### EXEMPLE 1

Une formulation cosmétique selon l'invention est préparée suivant la composition suivante :
- HUILE D'ARGAN........................40%
- ALOES ARBORESCENS...........40%
- BLANC DE BALEINE.................18%
- PEROX.d'HYD.....1%
- BORATE DE SODIUM............ 0,5%
- EXTRAIT DE PARFUM........... 0,5%
Cette formulation est appliquée sur la peau.
On observe après une semaine d'application quotidienne :
- une peau adoucie et réhydratée
- une diminution des rides
- une atténuation des taches de vieillesse.

## Revendications

1. Composition comprenant de l'huile d'argan et un produit à base de plante du genre *Aloe.*

2. Composition selon la revendication 1, **caractérisée en ce que** ledit produit à base de plante du genre *Aloe* est un jus de feuille d'une plante du genre *Aloe.*

3. Composition selon l'une quelconque des revendications 1 à 2, **caractérisée en ce que** ladite plante du genre *Aloe* est *l'Aloe arborescens.*

4. Composition selon l'une quelconque des revendications 1 à 3, **caractérisée en ce que** l'huile d'argan est présente à raison de 20% à 30% en poids de ladite composition

5. Composition selon l'une quelconque des revendications 1 à 4, **caractérisée en ce que** ledit produit à base de plante du genre *Aloe* est présent à raison de 20% à 30% en poids de ladite composition.

6. Utilisation d'une composition selon l'une quelconque des revendications 1 à 5 pour la préparation d'une formulation cosmétique.

7. Formulation cosmétique comprenant une composition selon l'une quelconque des revendications 1 à 5.

8. Formulation cosmétique selon la revendication 7, **caractérisée en ce que** ladite formulation est une lotion, une émulsion fluide, une crème, un lait, une mousse, un gel ou un patch.

9. Utilisation d'une formulation cosmétique selon l'une quelconque des revendications 7 à 8 en application topique pour nourrir, revitaliser et/ou raffermir la peau, les cheveux et/ou les ongles.

10. Utilisation d'une formulation cosmétique selon l'une quelconque des revendications 7 à 8 en application topique sur la peau à activité anti-âge et/ou anti-rides.

11. Utilisation d'une formulation cosmétique selon l'une quelconque des revendications 7 à 8 en application topique sur la peau pour atténuer les taches de vieillesse.
